# EUROPEAN PATENT APPLICATION

(11) **EP 4 830 748 A1**
(43) Date of publication of application: **28.10.2026**
(21) Application number: 25766863.2
(22) Date of filing: 08.04.2025
(51) Int. Cl.: A61M 5/315

(54) **SOUND PRODUCTION STRUCTURE FOR DOSE ADJUSTMENT, TORSION-SPRING AUTOINJECTOR, AND RATCHET TOOTH ASSEMBLY**

(30) Priority: 20.02.2025 CN 202510191187
(71) Applicant: Jiangsu Enjosim Medical Technology Co., Ltd, Wuxi, Jiangsu 214104 (CN)
(72) Inventor: CHEN, Feng, Wuxi, Jiangsu 214104 (CN)
(74) Representative: Bringer IP
(86) International application number: PCT/CN2025/087824
(87) International publication number: WO 2026/174645

(57) **Abstract**

A dosage adjustment sound-emitting structure, a torsion spring-based auto-injector, and a jump tooth member. The dosage adjustment sound-emitting structure includes a clutch tooth member, a jump tooth member, and a knob. A clutch ratchet tooth ring is disposed on an upper end surface of the clutch tooth member. The jump tooth member is disposed above the clutch tooth member and sheathed in the knob. A first component and a second component configured to fit with each other are respectively disposed on the jump tooth member and the knob. An elastic arm is disposed on the jump tooth member. A jump ratchet tooth meshing with the clutch ratchet tooth ring are disposed at a lower end of the elastic arm. An adjustment protruding block is disposed on a side wall of the knob. The adjustment protruding block is disposed below the elastic arm, and can jack up the elastic arm.

## Description

### TECHNICAL FIELD

The present invention relates to the technical field of medical devices, and specifically, to a dosage adjustment sound-emitting structure, an auto-injector, and a jump tooth structure.

### BACKGROUND

Auto-injectors can lower the operation difficulty of injectable therapy and reduce pain of patients, and therefore have been popular with medical workers and patients. All auto-injectors include an energy storage device, and energy stored by the energy storage device is released to inject a set dose of medicament into a user.

For a known wind-up pen-type injector using a torsion spring, reference can be made to Patent Publication No. CN103249441B. This solution includes: a housing for containing a syringe or cartridge of medicament; a rotary drive shaft for being rotated by an adjustable preset amount to cause expression of a corresponding amount of medicament from the syringe or cartridge; and a torsion drive spring, in one end region anchored relative to the drive shaft, and at its other end region, being secured to a fitting adapted to non-rotatably engage a seat on the housing. However, for this solution, in terms of the adjustment of injection dosage, if a user has set a higher dosage than is expected, it is difficult to reduce the set dosage. In addition, many injectors do not provide an audible prompt during the adjustment of injection dosage, and which is not user-friendly.

Patent Publication No. CN105682711B discloses a pen-type drug injection device, including: a housing; a dial member; a torsion spring fixed between the housing and the dial member such that energy is accumulated upon rotation of the dial member relative to the housing; a drive member engaging a lead screw; a first clutch rotationally coupling the drive member and the housing in a coupled state and allowing relative rotation between the drive member and the housing in a de-coupled state; and a second clutch rotationally coupling the drive member and the dial member in a coupled state and allowing relative clockwise and anti-clockwise rotation between the drive member and the dial member in a de-coupled state. The second clutch includes a ratchet clicker with teeth having in a clockwise direction and an anti-clockwise direction different ramped tooth angles such that the teeth are allowed to override each other in the de-coupled state of the second clutch with a different resistance in the clockwise direction and the anti-clockwise direction. Therefore, a user may stretch or loosen the torsion spring to set or correct a dose. This solution provides a drive mechanism which allows to easily reduce an already set dose. However, the dose button 15 in this solution is axially constrained to the clutch plate 16, and this may be achieved by a snap-on connection with the clutch plate 16. The clutch plate 16 is in splined engagement with the dial grip 14, and the drive member 8 is in splined engagement with the inner housing 5 The return spring 13 needs to act to maintain the engagement of the ratchet features between the drive member 8 and the clutch plate 16. In this case, the return spring 13 presses the clutch plate 16, and consequently, adjustment rotation of the clutch plate 16 requires a large force, affecting user experience.

### SUMMARY

An objective of the present invention is to resolve the foregoing defect of the existing technology. A first objective of the present invention is to provide a dosage adjustment sound-emitting structure that can freely adjust an injection dosage more smoothly, and a torsion spring-based auto-injector using the dosage adjustment sound-emitting structure. A second objective is to provide a jump tooth structure with a simple and convenient structure.

A technical solution used in the present invention for resolving the technical problem thereof is as follows: A dosage adjustment sound-emitting structure is provided, including a clutch tooth member, a jump tooth member, and a knob. The jump tooth member is of a one-way ratchet. The jump tooth member is disposed above the clutch tooth member, and the jump tooth member is sheathed in the knob. A first component and a second component configured to fit with each other are respectively disposed on the jump tooth member and the knob. An elastic arm is further disposed on the jump tooth member. A lower end of the elastic arm extends out of a lower end surface of the jump tooth member and is provided with a jump ratchet tooth. A clutch ratchet tooth ring fitting with the jump ratchet tooth is disposed on an upper end surface of the clutch tooth member. An adjustment protruding block is further disposed on a side wall of the knob, and the adjustment protruding block is disposed below the elastic arm. When the knob is forwardly rotated, the first component and the second component fit with each other, so that the knob and the jump tooth member synchronously rotate, and the jump ratchet tooth slides on the clutch ratchet tooth ring to adjust a dosage. When the knob is reversely rotated, the adjustment protruding block presses against the elastic arm, to separate the jump ratchet tooth from the clutch ratchet tooth ring. In this way, the jump tooth member can freely rotate reversely.

Further, the first component may be an engaging slot, an engaging block, or other structures having a contact surface, etc. on the jump tooth member. The second component may be an engaging block, an engaging slot, or other structures having a contact surface, etc. on the knob to fit with the jump tooth member. A principle of the first component and the second component is that the first component and the second component contact each other to form a connection, so as to transmit a rotational torque.

Further, a tooth shape of the jump ratchet tooth and a tooth shape of the clutch ratchet tooth ring each include a forward rotation surface which an inclined surface and a reverse rotation surface which a vertical surface. When the jump ratchet tooth completely meshes with the clutch ratchet tooth ring, the vertical reverse rotation surface prevents the jump tooth member from freely rotating reversely. When the knob is forwardly rotated, the first component fits with the second component, and the knob and the jump tooth member are synchronously rotated. During forward rotation, the jump ratchet tooth slides on the clutch ratchet tooth ring. Due to an elastic force of the elastic arm, the jump ratchet tooth, after meshing with one tooth of the clutch ratchet tooth ring, will enter into another groove. In this way, the jump ratchet tooth repeatedly meshes with the clutch ratchet tooth ring, and continuously jumps between teeth and grooves of the clutch ratchet tooth ring. Meshing and collision between the jump ratchet tooth and an end surface of the clutch ratchet tooth ring generates a clicking forward-rotation prompt sound of a high volume. During forward rotation, the jump tooth member drives an injection means connected to the jump tooth member to synchronously rotate forwardly, to accumulate energy. When the knob is reversely rotated, the jump ratchet tooth first maintains meshing with the clutch ratchet tooth ring. Because a rotation engaging block may be rotated in a rotation engaging slot by an angle, the adjustment protruding block presses against the elastic arm upward during reverse rotation. When the knob is reversely rotated by an angle, the lower end of the elastic arm is jacked up by the adjustment protruding block, so that the jump ratchet tooth is separated from the clutch ratchet tooth ring, and the clutch ratchet tooth ring no longer blocks the reverse rotation surface of the jump ratchet tooth. In this way, the jump tooth member may be freely rotated reversely due to the energy accumulated by the injection means connected to the jump tooth member. During reverse rotation, the jump ratchet tooth meshes with the clutch ratchet tooth ring again due to the elastic force of the elastic arm. During such repeated reverse rotation, collision between the jump ratchet tooth and the end surface of the clutch ratchet tooth ring also generates a clicking reverse-rotation prompt sound of a high volume. During reverse rotation, the energy of the injection means is consumed to drive the jump tooth member to synchronously rotate reversely, and a value of a set dosage is simultaneously reduced.

Further, a first positioning block and a second positioning block are disposed on the clutch tooth member. The first positioning block is an axially disposed rib. Radial positioning portions configured to limit radial rotation of the clutch tooth member are disposed on left and right sides of the first positioning block. A first axial positioning portion configured to limit axial movement of the clutch tooth member is disposed at a bottom of the first positioning block. The second positioning block is an elastic protruding block. A mounting portion is obliquely disposed at a bottom of the second positioning block. A second axial positioning portion configured to limit axial movement of the clutch tooth member is disposed on an upper end surface of the second positioning block. In this way, the clutch tooth member can be conveniently mounted to a pen body of an injector.

Further, the lower end surface of the jump tooth member corresponding to the rotation engaging slot is obliquely disposed, and an upper end surface of the rotation engaging block is also obliquely disposed. In this way, the jump tooth member can be conveniently moved downward and sheathed in the knob.

Further, a plurality of bumpy textures are provided on an outer side surface of the knob to facilitate rotation of the knob.

Further, a connection ratchet or a connection keyway is disposed on the jump tooth member, to connect to the injection means.

Further, an upper end surface of the adjustment protruding block is disposed in an arc shape to fit with a lower end surface of the elastic arm. In this way, fitting among the clutch tooth member, the jump tooth member, and the knob is more compact, mutual activities between structures are smoother, and user experience during use is improved.

This solution further discloses a torsion spring-based auto-injector, including a pen body. A medicament bottle, an injection means, and the foregoing dosage adjustment sound-emitting structure are mounted on the pen body. The injection means includes a release button, a clutch barrel, a small push rod, a release spring, a torsion spring, and a push-rod drive member. A clutch tooth member is fixedly mounted at an upper end of the pen body. A knob is rotatably mounted at the upper end of the pen body. The release button is mounted at an upper end of the clutch barrel. The release spring is disposed inside the knob. Two ends of the release spring respectively abut against the release button and the pen body. The release spring enables the clutch barrel to be positioned at a first axial clutch-barrel position. When a dosage is set, the clutch barrel is positioned at the first axial clutch-barrel position, and the upper end of the clutch barrel is connected to the jump tooth member and can be synchronously rotated with the jump tooth member and the torsion spring. The release button is pressed to push the clutch barrel to be positioned at a second axial clutch-barrel position. The upper end of the clutch barrel is separated from the jump tooth member and the torsion spring is released, to drive the clutch barrel to rotate. When the clutch barrel is rotated, the small push rod is driven to rotate, and the push-rod drive member converts a rotational torque of the small push rod into axial propulsion, to push and express injected medicament from the medicament bottle.

Further, a guide rod is disposed at a lower end of the release button. The guide rod extends into the upper end of the clutch barrel. The release button is upward and downward movably mounted at the upper end of the clutch barrel by using the guide rod. An upper end of the release button extends out of an upper end of the knob for convenient pressing. Anti-dropping buckles configured to fit with each other are disposed between the guide rod and the clutch barrel. When the clutch barrel is positioned at the first axial clutch-barrel position, the anti-dropping buckles between the guide rod and the clutch barrel contact and fit with each other. A release void is provided between a lower end surface of the release button and an upper end surface of the clutch barrel. The release button is pressed downward, so that the release button moves downward along the guide rod. The anti-dropping buckles between the guide rod and the clutch barrel are separated from each other. After the lower end surface of the release button is in contact with the upper end surface of the clutch barrel, the clutch barrel may be pushed to be positioned at the second axial clutch-barrel position. After the release button is released, the release spring pushes the release button to move upward. After the anti-dropping buckles between the guide rod and the clutch barrel are in contact and fit with each other, the clutch barrel is driven to be restored to be positioned at the first axial clutch-barrel position.

Further, the dosage adjustment sound-emitting structure further includes a scale barrel. An inner wall of the scale barrel is in an upward and downward sliding connection with an outer wall of the clutch barrel, and the outer wall of the scale barrel is in a helical sliding connection with an inner wall of the pen body. A plurality of guide rails and slide blocks configured to fit with each other are disposed on the inner wall of the scale barrel and the outer wall of the clutch barrel, and the guide rails and slide blocks are circularly disposed on the inner wall of the scale barrel and the outer wall of the clutch barrel uniformly. A zero-position limiting rib and a finish limiting rib that fit with the scale barrel are respectively disposed at an upper end and a lower end of the outer wall of the clutch barrel.

Further, in consideration of that some medicament bottles have a large amount of medicament and injection may be performed in a plurality of times, the injection means of this design further includes a large push rod. The push-rod drive member is mounted inside the pen body. A positioning block or positioning slot that fits with the pen body to avoid rotation of the push-rod drive member and facilitate mounting is disposed on a side wall of the push-rod drive member. A first drive ratchet is disposed on the push-rod drive member. First drive ratchet teeth that fit with the first drive ratchet are disposed at a lower end of the large push rod. The small push rod is provided with an external drive thread. The push-rod drive member is provided with an internal drive thread fitting with the small push rod. The push-rod drive member is in a threaded connection with the small push rod, a lower end of the small push rod passes through the large push rod and the push-rod drive member and is connected to a push handle and a rubber plug that facilitate pressing the medicament bottle. A ribbed slot fitting with the small push rod is disposed inside the large push rod. Second drive ratchet teeth are disposed on the large push rod. A second drive ratchet is disposed at a lower end of the clutch barrel. During dosage setting, the clutch barrel is positioned at the first axial clutch-barrel position, and a gap of approximately 0.2 mm is provided between the lower end of the clutch barrel and the large push rod. The second drive ratchet does not mesh with the second drive ratchet teeth. In this case, when the knob is forwardly or reversely rotated, the clutch barrel is forwardly or reversely rotated independently. In this case, the torsion spring may accumulate energy along with the forward rotation of the clutch barrel. During injection, the clutch barrel is positioned at the second axial clutch-barrel position, the lower end of the clutch barrel is connected to the large push rod, the second drive ratchet meshes with the second drive ratchet teeth, the accumulated energy of the torsion spring is released to drive the clutch barrel to rotate anticlockwise. In this case, the clutch barrel is already connected to the large push rod, to drive the large push rod to rotate anticlockwise, and the ribbed slot inside the large push rod drives the small push rod to helically move downward anticlockwise, to conduct the energy to the push handle and push the rubber plug for downward injection. After the injection is completed, the clutch barrel is restored to be positioned at the first axial clutch-barrel position, and the clutch barrel is separated from the large push rod. This process is repeated until the small push rod moves to the bottom and injection cannot be performed, so that injection of the entire bottle of medicament is completed. A flexible ratchet arm is disposed at the lower end of the large push rod. The first drive ratchet teeth are disposed on an outer end surface of the flexible ratchet arm. A stopping surface is provided on the first drive ratchet teeth, so that the large push rod can only be rotated relative to the push-rod drive member in one direction (anticlockwise). In this way, when the first drive ratchet and the first drive ratchet teeth are meshed and rotated, the flexible ratchet arm continuously jumps on a ratchet surface, and a clicking injection prompt sound is generated.

Further, the injection means further includes a memory member. An inner wall of the memory member is in a threaded connection with the small push rod. An outer wall of the memory member is in a sliding connection with an inner wall of the clutch barrel. A slide rail fitting with the memory member is disposed on the inner wall of the clutch barrel. The memory member is upward and downward movably mounted to the small push rod. An assembly portion fitting the large push rod is disposed on a lower end surface of the memory member. A stopping portion fitting an upper end surface of the memory member is disposed at a top of the small push rod. During dosage setting, the clutch barrel is positioned at the first axial clutch-barrel position, the lower end surface of the memory member fits the large push rod, the knob drives the jump tooth member and the clutch barrel to be rotated clockwise, and the clutch barrel drives the memory member to helically move upward along the small push rod. In this process, the torsion spring accumulates energy, the clutch barrel does not mesh with the large push rod, and the knob is twisted clockwise to a required scale. During injection, a top cap is pressed downward to release the torsion spring. During downward pressing, the clutch barrel meshes with the large push rod. After the meshing, the clutch barrel is separated from the jump tooth member, to release the energy accumulated by the torsion spring. When the torsion spring is restored, the torsion spring drives the large push rod to rotate anticlockwise, the large push rod drives the small push rod to helically move downward, to push the push handle for injection, and drive the memory member to be restored to a position at which the large push rod is assembled. An injection-complete audible prompt of "Pa" may be generated at a moment when the memory member is in contact with the large push rod. This process is repeated until the small push rod moves to the bottom and is stuck with the memory member, and injection cannot be performed, so that injection of the entire bottle of medicament is completed. In this case, the knob can no longer be rotated.

Further, the pen body includes an upper pen body and a lower pen body that are connected to each other. The lower pen body includes a refill holder and a pen cap. A cartridge is mounted inside the refill holder. An upper end of the refill holder is engaged to or in a threaded connection with the upper pen body. The pen cap is detachably sheathed in the refill holder. A pen clip is further disposed on the pen cap for ease of carrying.

This solution further discloses a jump tooth member that is a one-way ratchet and includes a tooth body. An elastic arm is disposed on the tooth body. The elastic arm is obliquely disposed downward. A jump ratchet tooth is disposed at a lower end of the elastic arm. The one-way ratchet uses a structure of the elastic arm that is obliquely disposed downward and the jump ratchet tooth, so that production, assembly, and application of the one-way ratchet are all convenient.

Further, a junction between an upper end of the elastic arm and the tooth body and a junction between the lower end of the elastic arm and the jump ratchet tooth are configured as arc transitions. In this way, the junctions of the elastic arm are not easily broken under excessive stress, achieving good fatigue resistance and a long service life.

In comparison with the existing technology, the present invention has the following beneficial effects. The dosage adjustment sound-emitting structure and the matching torsion spring-based auto-injector of this design have compact structures. The jump tooth member and the knob are engaged together, a fitting gap between the jump tooth member and the clutch tooth member is small, structures are compact, and a cooperation degree is high, so that an outline and a volume of an injector product can be greatly reduced, the knob, the clutch tooth member, and the jump tooth member may all be made of plastic, production and assembly are convenient, and a weight is low. The clutch tooth member and the jump tooth member fit with each other by using ratchet teeth upward and downward disposed axially, and meshing between the jump ratchet tooth and the clutch ratchet tooth ring is maintained by using the elastic force of the elastic arm, so that a smaller force is required for twisting the knob to adjust a dosage, thereby improving user experience of a user during use. The jump tooth member in this design uses the structure of the elastic arm that is obliquely disposed downward and the jump ratchet tooth, so that production, assembly, and application of the jump tooth member are all convenient.

### BRIEF DESCRIPTION OF THE DRAWINGS

FIG. 1 is an explosive diagram of a structure of a dosage adjustment sound-emitting structure according to the present invention;
FIG. 2 is a schematic cross-sectional diagram of an assembly structure of a dosage adjustment sound-emitting structure according to the present invention;
FIG. 3 is a schematic diagram of an assembly structure of a knob and a jump tooth member according to the present invention;
FIG. 4 and FIG. 5 are respectively a schematic diagram of a structure of a clutch tooth member and a schematic diagram of a structure of a jump tooth member according to the present invention;
FIG. 6 is a cross-sectional assembly diagram of a structure of a torsion spring-based auto-injector according to the present invention;
FIG. 7 is an assembly diagram of a structure of a small push rod according to the present invention;
FIG. 8 is a cross-sectional diagram of a structure of an upper pen body according to the present invention;
FIG. 9 is an assembly diagram of a structure of a push-rod drive member according to the present invention; and
FIG. 10 to FIG. 12 are respectively a schematic diagram of a structure of a push-rod drive member, a schematic diagram of a structure of a memory member, and a schematic diagram of a structure of a large push rod according to the present invention.

### DETAILED DESCRIPTION

The present invention is further described below with reference to the accompanying drawings and specific embodiments. It should be noted that the following specific embodiments are merely representative specific embodiments of the present invention, and a specific method, apparatus, condition, material, and the like described as examples are not intended to limit the present invention or the corresponding specific embodiments.

In addition, in this specification, terms "lower", "lower end", and "lower part" refer to one end in a direction of an injection needle, and "upper", "upper end", and "upper part" refer to an opposite direction, that is, one end in a direction of a release button.

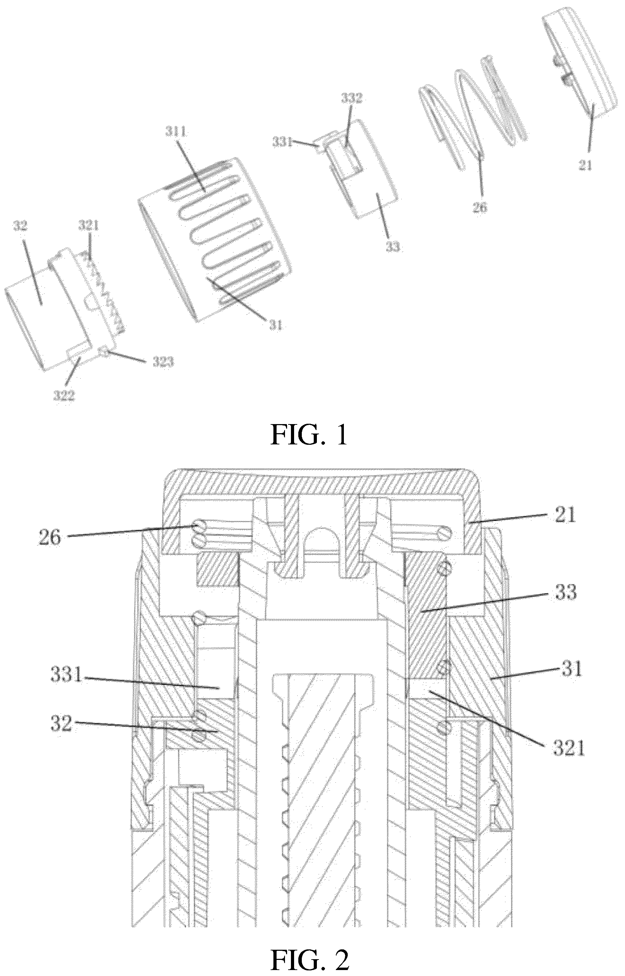

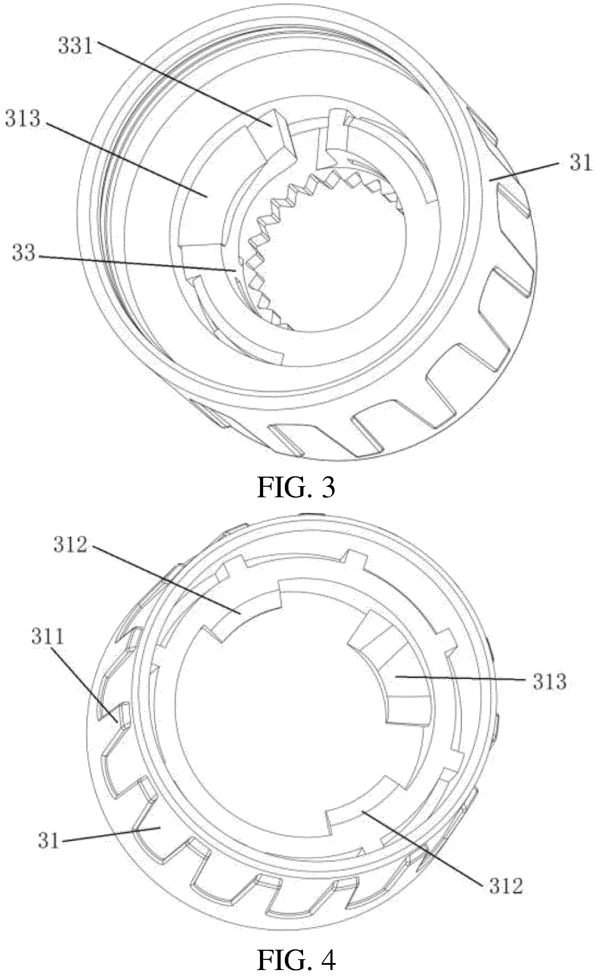

As shown in FIG. 1 to FIG. 5, a dosage adjustment sound-emitting structure is provided, including a knob 31, a clutch tooth member 32, and a jump tooth member 33. The jump tooth member 33 is of a one-way ratchet structure. A clutch ratchet tooth ring 321 is disposed on an upper end surface of the clutch tooth member 32. The jump tooth member 33 is disposed above the clutch tooth member 32, and the jump tooth member 33 is sheathed in the knob 31. A first component and a second component configured to fit with each other are respectively disposed on the jump tooth member 33 and the knob 31. The first component may be an engaging slot, an engaging block, or other structures having a contact surface, etc. on the jump tooth member 33. The second component may be an engaging block, an engaging slot, or other structures having a contact surface, etc. on the knob 31 to fit with the jump tooth member 33. A principle of the first component and the second component is that the first component and the second component contact each other to form a connection, so as to transmit a rotational torque. In this embodiment, descriptions are given using a specific embodiment in which a rotation engaging slot 332 and a rotation engaging block 312 configured to fit with each other are respectively disposed on the jump tooth member 33 and the knob 31. A forward rotation blocking portion and a reverse rotation blocking portion are respectively disposed at two ends of the rotation engaging slot 332. An elastic arm 333 is disposed on the jump tooth member 33. A lower end of the elastic arm 333 extends out of a lower end surface of the jump tooth member 33 and is provided with a jump ratchet tooth 331. The jump ratchet tooth 331 is configured to mesh with the clutch ratchet tooth ring 321. An adjustment protruding block 313 is further disposed on a side wall of the knob 31, and the adjustment protruding block 313 is disposed below the elastic arm 333. When the knob 31 is forwardly rotated, the forward rotation blocking portion blocks the rotation engaging block 312, so that the knob 31 and the jump tooth member 33 synchronously rotate, and the jump ratchet tooth 331 slides on the clutch ratchet tooth ring 321. When the knob 31 is reversely rotated, the adjustment protruding block 313 presses against the elastic arm 333, and the adjustment protruding block 313 jacks up the lower end of the elastic arm 333, to separate the jump ratchet tooth 331 from the clutch ratchet tooth ring 321. In this way, the jump tooth member 33 can freely rotate reversely.

Further, a tooth shape of the jump ratchet tooth 331 and a tooth shape of the clutch ratchet tooth ring 321 each include a forward rotation surface which is an inclined surface and a reverse rotation surface which is a vertical surface. When the jump ratchet tooth 331 completely meshes with the clutch ratchet tooth ring 321, the vertical reverse rotation surface prevents the jump tooth member from freely rotating reversely. When the knob 31 is forwardly rotated, the forward rotation blocking portion blocks the rotation engaging block 312, and the knob 31 and the jump tooth member 33 are synchronously rotated. During forward rotation, the jump ratchet tooth 331 slides on the clutch ratchet tooth ring 321 due to an elastic force of the elastic arm 333. Because the elastic arm is elastic, the jump ratchet tooth 331, after meshing with one tooth of the clutch ratchet tooth ring 321, will enter into another groove. In this way, the jump ratchet tooth 331 repeatedly meshes with the clutch ratchet tooth ring 321, and continuously jumps between teeth and grooves of the clutch ratchet tooth ring 321. Meshing and collision between the jump ratchet tooth 331 and an end surface of the clutch ratchet tooth ring 321 generates a clicking forward-rotation prompt sound of a high volume. During forward rotation, the jump tooth member 33 drives an injection means connected to the jump tooth member 33 to synchronously rotate forwardly, to accumulate energy and set a dosage. When the knob 31 is reversely rotated, the jump ratchet tooth 331 first maintains meshing with the clutch ratchet tooth ring 321. Because the rotation engaging block 312 may be rotated in the rotation engaging slot 332 by an angle, the adjustment protruding block 313 presses against the elastic arm 333 upward during reverse rotation. When the knob 31 is reversely rotated by an angle, the lower end of the elastic arm 333 is jacked up by the adjustment protruding block 313, so that the jump ratchet tooth 331 is separated from the clutch ratchet tooth ring 321. There is sufficient space between the reverse rotation blocking portion and the forward rotation blocking portion to allow the rotation engaging block 312 to be sufficiently rotated reversely, so that the adjustment protruding block 313 can jack up the elastic arm 333 to separate the jump ratchet tooth from the clutch ratchet tooth ring, and the clutch ratchet tooth ring 321 no longer blocks the reverse rotation surface of the jump ratchet tooth 331. In this way, the jump tooth member 33 may be freely rotated reversely due to the energy accumulated by the injection means connected to the jump tooth member. During reverse rotation, each time the knob 31 is reversely rotated by one tooth, the jump ratchet tooth 331 completely meshes with the clutch ratchet tooth ring 321 again due to the elastic force of the elastic arm, and as the knob 31 continues to be reversely rotated, the adjustment protruding block 313 jacks up the elastic arm 333. During such repeated reverse rotation, collision between the jump ratchet tooth 331 and the end surface of the clutch ratchet tooth ring 321 also generates a clicking reverse-rotation prompt sound of a high volume. During reverse rotation, the energy of the injection means is consumed to drive the jump tooth member to synchronously rotate reversely.

Further, a plurality of first positioning blocks 322 and a plurality of second positioning blocks 323 are disposed on the clutch tooth member 32. The first positioning blocks 322 are axially disposed ribs. Radial positioning portions configured to limit radial rotation of the clutch tooth member are disposed on left and right sides of the first positioning block 322. A first axial positioning portion configured to limit axial movement of the clutch tooth member is disposed at a bottom of the first positioning block 322. The second positioning blocks 323 are elastic protruding blocks. A mounting portion is obliquely disposed at a bottom of the second positioning block 323. A second axial positioning portion configured to limit axial movement of the clutch tooth member 32 is disposed on an upper end surface of the second positioning block 323. In this way, the clutch tooth member can be conveniently mounted to a pen body of an injector.

Further, the lower end surface of the jump tooth member 33 corresponding to the rotation engaging slot 332 is obliquely disposed, and an upper end surface of the rotation engaging block 312 is also obliquely disposed. In this way, the jump tooth member 33 can be conveniently moved downward and sheathed in the knob 31.

Further, a plurality of bumpy textures 311 are provided on an outer side surface of the knob 31 to facilitate rotation of the knob 31.

Further, the elastic arm 333 is obliquely disposed downward. A junction between an upper end of the elastic arm 333 and the jump tooth member 33 and a junction between the lower end of the elastic arm 333 and the jump ratchet tooth 331 are configured as arc transitions. In this way, the junctions of the elastic arm 333 are not easily broken under excessive stress, achieving good fatigue resistance and a long service life.

Further, a connection ratchet 334 or a connection keyway is disposed on the jump tooth member 33, to connect to the injection means.

Further, an upper end surface of the adjustment protruding block 313 is disposed in an arc shape to fit with a lower end surface of the elastic arm 333. In this way, cooperation among the clutch tooth member 32, the jump tooth member 33, and the knob 31 is more compact, mutual activities between structures are smoother, and user experience during use is improved.

The dosage adjustment sound-emitting structure of this embodiment is entirely light. The jump tooth member 33 and the knob 31 are engaged together, a fitting gap between the jump tooth member 33 and the clutch tooth member 32 is small, structures are compact, and a cooperation degree is high, so that an outline and a volume of an injector product can be greatly reduced, the knob 31, the clutch tooth member 32, and the jump tooth member 33 may all be made of plastic, and production and assembly are convenient. The clutch tooth member 32 and the jump tooth member 33 fit with each other by using ratchet teeth upward and downward disposed axially, and meshing between the jump ratchet tooth and the clutch ratchet tooth ring is maintained by using the elastic force of the elastic arm, so that a small force is required for twisting the knob 31 to adjust a dosage, and user experience of a user during use is good.

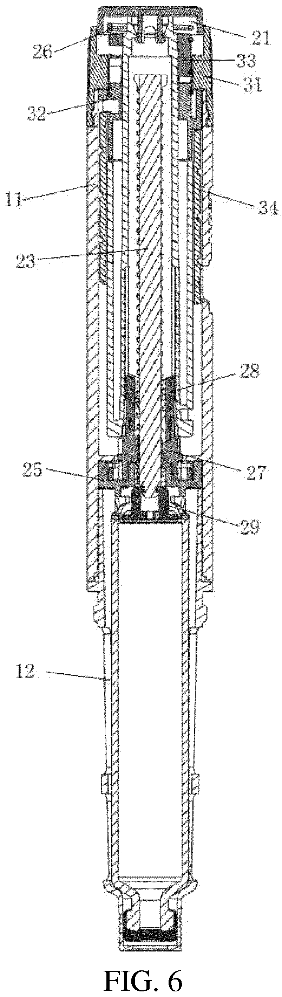

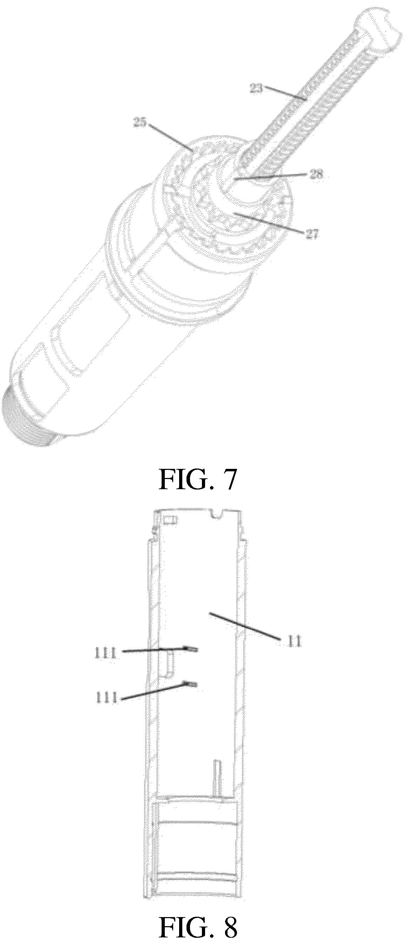

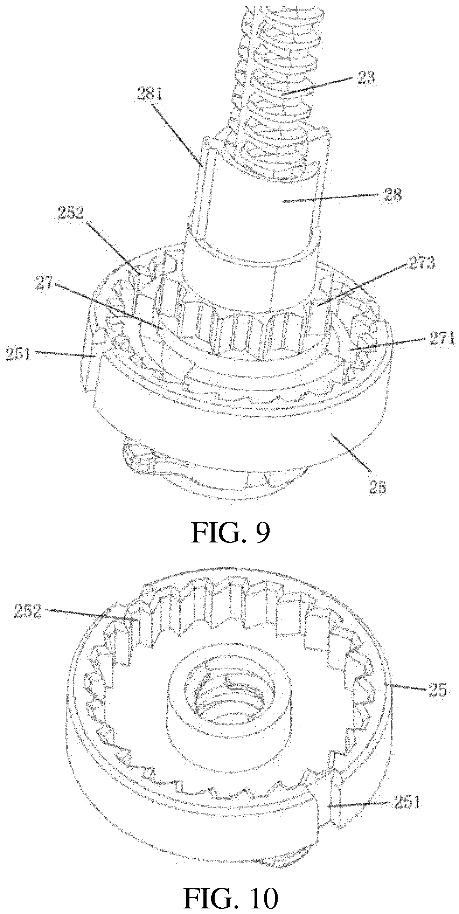

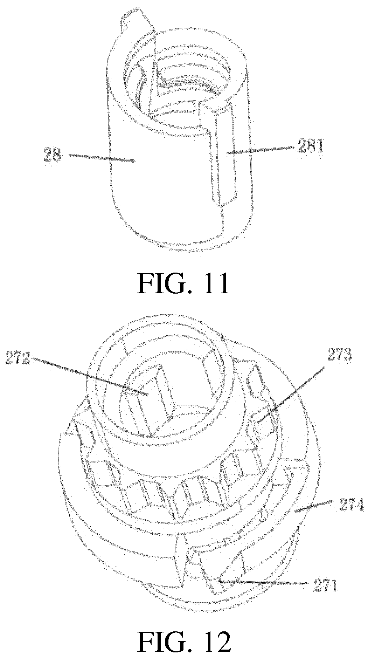

This embodiment further discloses a torsion spring-based auto-injector in which the foregoing dosage adjustment sound-emitting structure is used. As shown in FIG. 6 to FIG. 12, the torsion spring-based auto-injector includes a pen body. The pen body includes an upper pen body 11 and a lower pen body that are connected to each other. The lower pen body includes a refill holder 12 and a pen cap. A cartridge is mounted inside the refill holder 12. An upper end of the refill holder 12 is engaged to or in a threaded connection with the upper pen body 11. A connection thread connected to an injection needle is disposed at a lower end of the refill holder 12. The pen cap is detachably sheathed in the refill holder 12. Elastic buckles configured to fit with each other may be disposed between the refill holder 12 and the pen cap for positioning. An injection means and a dosage adjustment sound-emitting structure are mounted to the upper pen body 11.

The injection means includes a release button 21, a clutch barrel 22, a small push rod 23, a torsion spring 24, a push-rod drive member 25, a release spring 26, a large push rod 27, and a memory member 28. The dosage adjustment sound-emitting structure includes a knob 31, a clutch tooth member 32, and a jump tooth member 33. The knob 31 is rotatably mounted at the upper end of the upper pen body 11. The clutch tooth member 32 are engaged to the upper pen body 11 and cannot be rotated. The jump tooth member 33 is engaged and disposed on an inner side of the knob 31. A clutch ratchet tooth ring 321 is disposed on an upper end surface of the clutch tooth member 32. An elastic arm 333 is disposed on the jump tooth member 33. A lower end of the elastic arm 333 extends out of a lower end surface of the jump tooth member 33 and is provided with a jump ratchet tooth 331 meshing with the clutch ratchet tooth ring 321. An upper end of the clutch barrel 22 passes through the clutch tooth member 32 and the jump tooth member 33. The upper end of the clutch barrel 22 is connected to the jump tooth member 33 by using a ratchet (where a key connection manner may alternatively be used in another embodiment) to implement synchronous rotation. The clutch barrel 22 and the jump tooth member 33 can upward and downward move axially to implement separation. A guide rod 211 is disposed at a lower end of the release button 21. The guide rod 211 extends into the upper end of the clutch barrel 22. The release button 21 is upward and downward movably mounted at the upper end of the clutch barrel 22 by using the guide rod 211. An upper end of the release button 21 extends out of an upper end of the knob 31 for convenient pressing. Anti-dropping buckles configured to fit with each other are disposed between the guide rod 211 and the clutch barrel 22. The release spring 26 is disposed inside the knob 31. Two ends of the release spring 26 respectively abut against the release button 21 and the clutch tooth member 32 (which may alternatively be the knob 31 or the pen body). The release spring 26 provides a spring force to maintain the clutch barrel 22 at a first axial clutch-barrel position. The push-rod drive member 25 is mounted inside a lower end of the upper pen body 11. A positioning block or positioning slot 251 configured to fit with the upper pen body 11 to avoid rotation of the push-rod drive member 25 and facilitate mounting is arranged on a side wall of the push-rod drive member 25. A groove is provided in the middle of the push-rod drive member 25. A first drive ratchet 252 is disposed on an inner wall of the groove. A lower end of the large push rod 27 is carried and disposed on an end surface of the groove. First drive ratchet teeth 271 that fit with the first drive ratchet 252 are disposed on a side carrier wall at the lower end of the large push rod 27. The small push rod 23 is provided with an external drive thread. The push-rod drive member 25 is provided with an internal drive thread fitting with the small push rod 23. The push-rod drive member 25 is in a threaded connection with the small push rod 23, a lower end of the small push rod 23 passes through the large push rod 27 and the push-rod drive member 25 and is connected to a push handle and a rubber plug that facilitate pressing the medicament bottle. A ribbed slot 272 fitting with the small push rod 23 is disposed inside the large push rod 27. Second drive ratchet teeth 273 are further disposed on the large push rod 27. A second drive ratchet 221 fitting with the second drive ratchet teeth 273 is disposed at a lower end of the clutch barrel 22. An inner wall of the memory member 28 is in a threaded connection with the small push rod 23. An outer wall of the memory member 28 is in a sliding connection with an inner wall of the clutch barrel 22. A slide-block rib 281 is disposed on the outer wall of the memory member 28. A slide rail fitting with the slide-block rib 281 is disposed on the inner wall of the clutch barrel 22. The memory member 28 is upward and downward movably mounted to the small push rod 23. An assembly portion fitting the large push rod 27 is disposed on a lower end surface of the memory member 28. A stopping portion fitting an upper end surface of the memory member 28 is disposed at a top of the small push rod 23.

During dosage setting, the clutch barrel 22 is positioned at the first axial clutch-barrel position, the anti-dropping buckles between the guide rod 211 and the clutch barrel 22 are in contact and fit with each other, and a release void is provided between a lower end surface of the release button 21 and an upper end surface of the clutch barrel 22 A gap of approximately 0.2 mm is provided between the lower end of the clutch barrel 22 and the large push rod 27. The second drive ratchet 221 does not mesh with the second drive ratchet teeth 273. In this case, when the knob 31 is forwardly or reversely rotated, the clutch barrel 22 is forwardly or reversely rotated independently. In this case, the torsion spring may accumulate energy along with the forward rotation of the clutch barrel 22. In this case, the lower end surface of the memory member 28 fits an upper end surface of the large push rod 27. When the knob 31 drives, by using the rotation engaging block 312, the jump tooth member 33 to rotate forwardly, the jump ratchet tooth 331 slides on the clutch ratchet tooth ring 321, the upper end of the clutch barrel 22 is connected to the jump tooth member 33 and can be synchronously rotated with the jump tooth member 33 and the torsion spring 24. The clutch barrel 22 drives the memory member 28 to move upward along the small push rod 23. In this process, the torsion spring 24 accumulates energy. When a dosage expected to be set is reached, rotation of the knob is stopped, the jump ratchet tooth 331 completely meshes with the clutch ratchet tooth ring 321, and the vertical reverse rotation surface of the jump ratchet tooth 331 and the clutch ratchet tooth ring 321 prevents the jump tooth member from freely rotating reversely When the knob is reversely rotated, when the jump ratchet tooth 331 is separated from the clutch ratchet tooth ring 321, an effect of limiting the jump ratchet tooth 331 by the clutch ratchet tooth ring 321 disappears, the energy accumulated by the torsion spring 34 can actively drive the clutch barrel 22 and the jump tooth member 33 to synchronously rotate reversely, so that the energy is consumed. When the clutch barrel 22 is rotated, the memory member 28 may also be driven to move downward along the small push rod 23.

The release button 21 is pressed, so that the release button 21 moves downward along the guide rod 211. The anti-dropping buckles between the guide rod 211 and the clutch barrel 22 are separated from each other. After the lower end surface of the release button 21 is in contact with the upper end surface of the clutch barrel 22, the clutch barrel 22 may be pushed to be positioned at the second axial clutch-barrel position, the upper end of the clutch barrel 22 is separated from the jump tooth member 33, the lower end of the clutch barrel 22 is connected to the large push rod 27, and the second drive ratchet meshes with the second drive ratchet teeth. In this case, the accumulated energy of the torsion spring 24 is released to drive the clutch barrel 22 to rotate anticlockwise. In this case, the clutch barrel is already connected to the large push rod 27, to drive the large push rod 27 to rotate anticlockwise, and the ribbed slot inside the large push rod 27 drives the small push rod 23 to helically move downward anticlockwise, to conduct the energy to the push handle 25 and push the rubber plug for downward injection, so that injected medicament is pushed and expressed from the medicament bottle. When helically moving downward anticlockwise, the small push rod 23 drives the memory member 28 to be restored to a position at which the large push rod 27 is assembled, and an injection-complete audible prompt of "Pa" may be generated at a moment when the memory member 28 is in contact with the large push rod 27.

After the injection is completed, the release button 21 is released. Due to the release spring 26, after the anti-dropping buckles between the guide rod 211 and the clutch barrel 22 contact and fit with each other, the clutch barrel 22 is restored to be positioned at the first axial clutch-barrel position.

The position of the small push rod 23 on the push-rod drive member 25 is maintained as the small push rod 23 was at the time of completion of the injection, and all the other components are restored to positions where they were at the time of dosage setting. In this way, dosage setting and injection are repeated for a plurality of times until the small push rod 23 moves to the bottom and is stuck with the memory member 28, and injection cannot be performed, so that injection of the entire bottle of medicament is completed. In this case, the knob 31 can no longer be rotated.

Further, the dosage adjustment sound-emitting structure further includes a scale barrel 34. An inner wall of the scale barrel 34 is in an upward and downward sliding connection with an outer wall of the clutch barrel 22. A helical slide rail is disposed on an outer wall of the scale barrel 34. An engaging block fitting with the slide rail is disposed on an inner wall of the upper pen body 11. The outer wall of the scale barrel 34 is in a helical sliding connection with the inner wall of the upper pen body. In this way, when the clutch barrel 22 drives the scale barrel 34 to synchronously rotate, the scale barrel 34 is continuously rotated upward and downward under synchronous action of the outer wall and the inner wall, and a set injection amount can be accurately determined in cooperation with a window on the upper pen body 11. A plurality of guide rails and slide blocks configured to fit with each other are disposed on the inner wall of the scale barrel 34 and the outer wall of the clutch barrel 22, and the guide rails and slide blocks are circularly disposed on the inner wall of the scale barrel 34 and the outer wall of the clutch barrel 22 uniformly. A zero-position limiting rib and a finish limiting rib that fit with the scale barrel 34 are respectively disposed at an upper end and a lower end of the outer wall of the clutch barrel 22.

Further, a flexible ratchet arm 274 is disposed at the lower end of the large push rod 27. The first drive ratchet teeth 271 are disposed on an outer end surface of the flexible ratchet arm 274. A stopping surface is provided on the first drive ratchet teeth 271, so that the large push rod can only be rotated relative to the push-rod drive member 25 in one direction (anticlockwise). In this way, when the first drive ratchet 252 and the first drive ratchet teeth 271 are meshed and rotated, the flexible ratchet arm 274 continuously jumps on a ratchet surface, and a clicking injection prompt sound is generated.

Further, a pen clip is further disposed on the pen cap for ease of carrying.

A structure of the torsion spring-based auto-injector of this embodiment is simple, production and mounting are convenient, and it is easy to operate and carry the torsion spring-based auto-injector. An entire structure of the torsion spring-based auto-injector compactly and scientifically matches and has smooth actions. Injection is performed by using the torsion spring as a driving force, so that convenience and comfort of medicine are improved, and an injection process can be rapidly and stably completed, which is suitable for popularization and application. The dosage adjustment sound-emitting structure thereof uses the knob, the clutch tooth member, and the jump tooth member, and an expected dosage is obtained through adjustment by forwardly or reversely rotating the knob. Because the elastic force of the torsion spring is stable and controllable, the dosage adjustment sound-emitting structure can implement high-accuracy dosage setting, thereby ensuring accuracy of an injection dosage. In addition, an elastic property of the torsion spring enables the injection pen to be more stable during use, thereby reducing an injection deviation caused by interference from an external force. The elastic property of the torsion spring also enables the dosage adjustment sound-emitting structure to be automatically restored after use, thereby improving reliability and a service life of the injection pen. During forward or reverse rotation, the jump tooth member and the clutch tooth member fit to generate the audible prompt in an adjustment process, to facilitate use by a user. The clutch barrel, the large push rod, and the push-rod drive member of the injection means thereof are clutched by using internal teeth. The clutch barrel does not mesh with the large push rod when the injection means is not released, and a rotation and twisting operation during dosage setting is not affected. Combination of the large push rod, the memory member, and the push-rod drive member enables an injector to perform injection for a plurality of times, so that injection of the entire bottle of medicament is completed. In addition, the audible prompt may also be generated during injection, thereby facilitating use by the user.

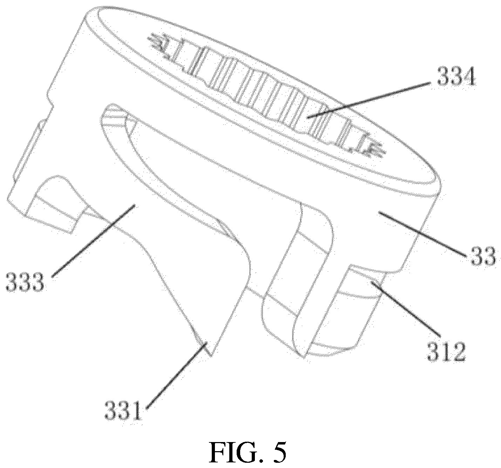

As shown in FIG. 5, this embodiment further discloses a jump tooth structure, including a tooth body. The tooth body is disposed in a tube shape. An inner diameter of an upper end of an inner wall of the tooth body is smaller than an inner diameter of a lower end of the inner wall of the tooth body. The upper end of the inner wall of the tooth body is used to provide a connection portion for connecting a drive member. A hollow portion is disposed at the lower end of the tooth body. An annular elastic arm 333 that rotates around a circle center is disposed at the hollow portion. The elastic arm 333 is obliquely disposed downward, and a jump ratchet tooth 331 is disposed at a lower end of the elastic arm 333. A junction between an upper end of the elastic arm 333 and the tooth body and a junction between the lower end of the elastic arm 333 and the jump ratchet tooth 331 are configured as arc transitions. In this way, the junctions of the elastic arm 333 are not easily broken under excessive stress, achieving good fatigue resistance and a long service life. The one-way ratchet uses a structure of the elastic arm that is obliquely disposed downward and the jump ratchet tooth, and may be integrally formed through injection molding by using a plastic material, so that production, assembly, and application of the one-way ratchet are all convenient. The elastic arm 333 is usually strip-shaped or arc-shaped, has elasticity and flexibility, and can be bent or deformed to adapt to different use requirements. This design enables the elastic arm 333 to be deformed when an external force is applied, thereby achieving effects of buffering, fixing, or regulation.

The foregoing descriptions are merely exemplary embodiments of the present invention, and certainly are not intended to limit the scope of implementation of the present invention. To be specific, all simple equivalent variations and modifications generally made in accordance with the scope of the present invention and the content of the descriptions of the present invention shall fall within the scope of the present invention.

## Claims

1. A dosage adjustment sound-emitting structure, comprising a clutch tooth member, a jump tooth member, and a knob, wherein the jump tooth member is disposed above the clutch tooth member, the jump tooth member is sheathed in the knob, and a first component and a second component configured to fit with each other are respectively disposed on the jump tooth member and the knob; an elastic arm is further disposed on the jump tooth member, a lower end of the elastic arm extends out of a lower end surface of the jump tooth member and is provided with a jump ratchet tooth, a clutch ratchet tooth ring fitting with the jump ratchet tooth is disposed on an upper end surface of the clutch tooth member, an adjustment protruding block is further disposed on a side wall of the knob, and the adjustment protruding block is disposed below the elastic arm; and when the knob is forwardly rotated, the first component and the second component fit with each other, to enable the knob and the jump tooth member to synchronously rotate, and the jump ratchet tooth slides on the clutch ratchet tooth ring to adjust a dosage; or when the knob is reversely rotated, the adjustment protruding block presses against the elastic arm, to separate the jump ratchet tooth from the clutch ratchet tooth ring.

2. The dosage adjustment sound-emitting structure according to claim 1, wherein a tooth shape of the jump ratchet tooth and a tooth shape of the clutch ratchet tooth ring each comprise a forward rotation surface which is an inclined surface and a reverse rotation surface which is a vertical surface.

3. The dosage adjustment sound-emitting structure according to claim 1 or 2, wherein a first positioning block and a second positioning block are disposed on the clutch tooth member, radial positioning portions configured to limit radial rotation of the clutch tooth member are disposed on left and right sides of the first positioning block, a first axial positioning portion configured to limit axial movement of the clutch tooth member is disposed at a bottom of the first positioning block, a mounting portion is obliquely disposed at a bottom of the second positioning block, and a second axial positioning portion configured to limit axial movement of the clutch tooth member is disposed on an upper end surface of the second positioning block.

4. The dosage adjustment sound-emitting structure according to claim 1, wherein a plurality of bumpy textures are provided on an outer side surface of the knob.

5. The dosage adjustment sound-emitting structure according to claim 1, wherein a connection ratchet or a connection keyway is disposed on the jump tooth member.

6. The dosage adjustment sound-emitting structure according to claim 1, wherein the lower end surface of the jump tooth member corresponding to a rotation engaging slot is obliquely disposed, and an upper end surface of a rotation engaging block is also obliquely disposed.

7. The dosage adjustment sound-emitting structure according to claim 1, wherein an upper end surface of the adjustment protruding block is disposed in an arc shape to fit with a lower end surface of the elastic arm.

8. A torsion spring-based auto-injector, comprising a pen body, wherein a medicament bottle, an injection means, and the dosage adjustment sound-emitting structure according to any one of claims 1 to 7 are mounted on the pen body, the injection means comprises a release button, a clutch barrel, a small push rod, a release spring, a torsion spring, and a push-rod drive member, a clutch tooth member is fixedly mounted at an upper end of the pen body, a knob is rotatably mounted at the upper end of the pen body, the release button is mounted at an upper end of the clutch barrel, the release spring is disposed inside the knob, two ends of the release spring respectively abut against the release button and the pen body, and the release spring enables the clutch barrel to be positioned at a first axial clutch-barrel position; when a dosage is set, the clutch barrel is positioned at the first axial clutch-barrel position, and the upper end of the clutch barrel is connected to the jump tooth member and can be synchronously rotated with the jump tooth member and the torsion spring; and the release button is pressed to push the clutch barrel to be positioned at a second axial clutch-barrel position, the upper end of the clutch barrel is separated from the jump tooth member and the torsion spring is released, to drive the clutch barrel to rotate, and when the clutch barrel is rotated, the small push rod is driven to rotate, and the push-rod drive member converts a rotational torque of the small push rod into axial propulsion, to push and express injected medicament from the medicament bottle.

9. The torsion spring-based auto-injector according to claim 8, wherein the injection means further comprises a large push rod, the push-rod drive member is mounted inside the pen body, a positioning block or positioning slot that fits with the pen body to avoid rotation of the push-rod drive member and facilitate mounting is disposed on a side wall of the push-rod drive member, a first drive ratchet is disposed on the push-rod drive member, first drive ratchet teeth that fit with the first drive ratchet are disposed at a lower end of the large push rod, the small push rod is provided with an external drive thread, the push-rod drive member is provided with an internal drive thread fitting with the small push rod, the push-rod drive member is in a threaded connection with the small push rod, a lower end of the small push rod passes through the large push rod and the push-rod drive member and is connected to a push handle and a rubber plug that facilitate pressing the medicament bottle, a ribbed slot fitting with the small push rod is disposed inside the large push rod, second drive ratchet teeth are disposed on the large push rod, a second drive ratchet is disposed at a lower end of the clutch barrel, and during dosage setting, the clutch barrel is positioned at the first axial clutch-barrel position, and a gap is provided between the lower end of the clutch barrel and the large push rod; and during injection, the clutch barrel is positioned at the second axial clutch-barrel position, the lower end of the clutch barrel is connected to the large push rod, the second drive ratchet meshes with the second drive ratchet teeth, accumulated energy of the torsion spring is released to drive the clutch barrel to rotate anticlockwise, the clutch barrel is already connected to the large push rod in this case, to drive the large push rod to rotate anticlockwise, and the ribbed slot inside the large push rod drives the small push rod to helically move downward anticlockwise, to conduct the energy to the push handle and push the rubber plug for downward injection.

10. A jump tooth member, comprising a tooth body, wherein an elastic arm is disposed on the tooth body, the elastic arm is obliquely disposed downward, and a jump ratchet tooth is disposed at a lower end of the elastic arm.
